Europäisches Patentamt

European Patent Office

Office européen des brevets

(19)

(11) Numéro de publication : **0 494 825 A1**

(12) # DEMANDE DE BREVET EUROPEEN

(21) Numéro de dépôt : **92400053.2**

(22) Date de dépôt : **09.01.92**

(51) Int. Cl.⁵ : **C07K 7/10, C12P 21/08,**
    **A61K 39/29, G01N 33/576**

(30) Priorité : **11.01.91 FR 9100273**

(43) Date de publication de la demande :
    **15.07.92 Bulletin 92/29**

(84) Etats contractants désignés :
    **AT BE CH DE DK ES FR GB GR IT LI LU MC NL
    PT SE**

(71) Demandeur : **CLONATEC S.A.
    60, Rue de Wattignies
    F-75580 Paris Cédex 12 (FR)**

(72) Inventeur : **Somme, Gérard
    2 Domaine du Vaularon
    F-91440 Bures-Sur-Yvette (FR)**

Inventeur : **Van Rietschoten, Jurphane
Val de la Torse - A2
F-13100 Aix-en-Provence (FR)**
Inventeur : **Sabatier, Jean-Marc
5, Chemin Neuf
F-13790 Rousset (FR)**
Inventeur : **Trepo, Christian
4, Passage du Verdier Sud
F-69500 Bron (FR)**
Inventeur : **Pichoud, Christian
3, Chemin de la Ferme
F-69120 Vaux-en-Velin (FR)**
Inventeur : **Martin, Jacques
155, Rue d'Alésia
F-75014 Paris (FR)**
Inventeur : **Tordjeman, Marc
5, Rue Eugène Jumin
F-75019 Paris (FR)**

(74) Mandataire : **Ores, Irène et al
CABINET ORES 6, Avenue de Messine
F-75008 Paris (FR)**

(54) **Peptides synthétiques dérivant de l'antigène HBc du virus de l'hépatite B.**

(57)   La présente invention concerne des peptides synthétiques constitués par tout ou partie d'au moins une des séquences peptidiques choisie parmi les séquences suivantes :

```
Leu-Tyr-Arg-Glu-Ala-Leu-Glu-Ser-Pro-Glu-His-Cys-Ser-Pro-
His-His-Thr-Ala-Leu-Arg-Gln-Ala

Val-Asn-Leu-Glu-Asp-Pro--Ala-Ser-Arg-Asp-Leu-Val-Val-Ser-
Tyr-Val-Asn-Thr-Asn-Met-Gly-Leu-Lys-Phe-Arg-Gln-Leu

Lys-Phe-Arg-Gln-Leu-Leu-Trp-Phe-His-Ile-Ser-Cys-Leu-Thr-
Phe-Gly-Arg-Glu-Thr-Val-Ile-Glu-Tyr

Gly-Arg-Ser-Pro-Arg-Arg-Arg-Thr-Pro-Ser-Pro-Arg-Arg-Arg-
Arg-Ser-Gln-Ser-Pro-Arg-Arg-Arg-Ser-Gln-Ser-Arg-Glu-
Ser-Gln-Cys

Val-Asn-Leu-Glu-Asp-Pro-Ala-Ser-Arg-Asp-Leu-Val-Val-Ser-
Tyr-Val-Asn-Thr-Asn-Met-Gly-Leu-Lys-Phe-Arg-Gln-Leu-Leu-
Trp-Phe-His-Ile-Ser-Cys-Leu-Thr-Phe-Gly-Arg-Glu-Thr-Val-
Ile-Glu-Tyr
```

Application au diagnostic et au vaccin.

EP 0 494 825 A1

La présente invention concerne des peptides ayant des propriétés immunologiques, le cas échéant immunogènes, en commun avec l'antigène HBc du virus de l'hépatite B.

L'hépatite B est une maladie virale fréquente, particulièrement dans les pays d'Afrique ou d'Extrême-Orient. L'infection se traduit souvent par une forme aiguë sans lendemain, mais peut être aussi à l'origine d'une hépatite chronique ou d'une cirrhose. La particule de Dane est considérée comme l'agent viral étiologique, elle possède une enveloppe externe dénommée antigène HBs et une nucléocapside interne dénommée antigène HBc.

Le génome du virus de l'hépatite B a été séquencé et cloné et les gènes codant pour les antigènes HBs et HBc ont été identifiés et ont permis de déduire les polypeptides codés par ces séquences génétiques. Le Brevet Européen N° 79303017.2 publié sous le N° 13 828 décrit notamment des fragments d'ADN codant pour des polypeptides ayant des propriétés immunologiques en commun avec les antigènes du virus de l'hépatite B.

L'antigène HBc est très fortement immunogène; lors d'une infection par le virus de l'hépatite B, il provoque une réponse immunitaire très forte et les anticorps qui en résultent peuvent persister des années après la primo-infection.

La demanderesse a mis à profit ses connaissances sur la synthèse de peptides pour préparer des peptides synthétiques mimant l'antigénicité HBc, pour la détection des anticorps anti-HBc présents dans les sérums ou plasma d'individus infectés par le virus de l'hépatite B.

L'invention concerne donc également l'application de ces peptides à la fabrication de compositions pour le diagnostic *in vitro* chez l'homme, d'une infection par le virus de l'hépatite B.

L'invention concerne en outre des peptides immunogènes ou susceptibles d'être rendus immunogènes *in vivo*, et l'application de ces peptides à la production de compositions immunogènes et de compositions vaccinantes contre le virus de l'hépatite B

L'invention concerne enfin les anticorps susceptibles d'être induits *in vivo* par les peptides immunogènes ou rendus immunogènes et leurs applications notamment à la production de principes actifs de médicaments contre une infection par le virus de l'hépatite B.

Pour désigner ci-après les résidus d'aminoacides entrant dans la constitution des peptides selon l'invention, on aura recours aux Normes IUPAC-IUB figurant dans NUCLEIC ACIDS RESEARCH 13, 3021-3030 (1985) et dans THE BIOCHEMICAL JOURNAL, 219, N° 2, 345-373 (1984).

Les peptides synthétiques de l'invention sont constitués par tout ou partie d'au moins une des séquences peptidiques choisie parmi les séquences suivantes :

```
Leu-Tyr-Arg-Glu-Ala-Leu-Glu-Ser-Pro-Glu-His-Cys-Ser-Pro-
His-His-Thr-Ala-Leu-Arg-Gln-Ala

Val-Asn-Leu-Glu-Asp-Pro--Ala-Ser-Arg-Asp-Leu-Val-Val-Ser-
Tyr-Val-Asn-Thr-Asn-Met-Gly-Leu-Lys-Phe-Arg-Gln-Leu

Lys-Phe-Arg-Gln-Leu-Leu-Trp-Phe-His-Ile-Ser-Cys-Leu-Thr-
Phe-Gly-Arg-Glu-Thr-Val-Ile-Glu-Tyr

Gly-Arg-Ser-Pro-Arg-Arg-Arg-Thr-Pro-Ser-Pro-Arg-Arg-Arg-
Arg-Ser-Gln-Ser-Pro-Arg-Arg-Arg-Arg-Ser-Gln-Ser-Arg-Glu-
Ser-Gln-Cys

Val-Asn-Leu-Glu-Asp-Pro-Ala-Ser-Arg-Asp-Leu-Val-Val-Ser-
Tyr-Val-Asn-Thr-Asn-Met-Gly-Leu-Lys-Phe-Arg-Gln-Leu-Leu-

Trp-Phe-His-Ile-Ser-Cys-Leu-Thr-Phe-Gly-Arg-Glu-Thr-Val-
Ile-Glu-Tyr
```

L'invention envisage à titre spécifique les peptides répondant aux formules suivantes :

```
Leu-Tyr-Arg-Glu-Ala-Leu-Glu-Ser-Pro-Glu-His-Cys-Ser-Pro-
His-His-Thr-Ala-Leu-Arg-Gln-Ala                    (I)
Val-Asn-Leu-Glu-Asp-Pro--Ala-Ser-Arg-Asp-Leu-Val-Val-Ser-
Tyr-Val-Asn-Thr-Asn-Met-Gly-Leu-Lys-Phe-Arg-Gln-Leu (II)
Lys-Phe-Arg-Gln-Leu-Leu-Trp-Phe-His-Ile-Ser-Cys-Leu-Thr-
Phe-Gly-Arg-Glu-Thr-Val-Ile-Glu-Tyr                (III)
Gly-Arg-Ser-Pro-Arg-Arg-Arg-Thr-Pro-Ser-Pro-Arg-Arg-
Arg-Ser-Gln-Ser-Pro-Arg-Arg-Arg-Arg-Ser-Gln-Ser-Arg-Glu-
Ser-Gln-Cys                                        (IV)
Val-Asn-Leu-Glu-Asp-Pro-Ala-Ser-Arg-Asp-Leu-Val-Val-Ser-
Tyr-Val-Asn-Thr-Asn-Met-Gly-Leu-Lys-Phe-Arg-Gln-Leu-Leu-
Trp-Phe-His-Ile-Ser-Cys-Leu-Thr-Phe-Gly-Arg-Glu-Thr-Val-
Ile-Glu-Tyr                                        (V)
```

Les peptides, selon l'invention, sont préparés par synthèse peptidique en phase solide soit par condensation successive des résidus d'acides aminés dans l'ordre requis, soit par condensation de résidus d'acides aminés sur un fragment préalablement formé contenant déjà plusieurs acides aminés dans l'ordre approprié ou encore par condensation de plusieurs fragments préalablement préparés, en prenant soin de protéger au préalable, toutes les fonctions réactives portées par les résidus d'acides aminés ou les fragments, excepté les fonctions amines et carboxyles engagées dans la liaison peptidique formée lors de la condensation.

Selon un exemple de préparation du peptide de formule (IV), on fixe sur une résine, par l'intermédiaire de son groupe carboxylique le résidu Cys dont la fonction amine est protégée par un groupe terbutyloxycarbonyl, puis après avoir déprotégé la fonction amine en lavant la résine avec de l'acide trifluoroacétique dans du dichlorométhane, on couple en présence de diméthylformamide ou de N-méthylpyrrolidinone le second résidu d'acide aminé Gln dont la fonction amine est protégée comme précédemment, on fixe ainsi les uns après les autres les résidus d'acides aminés. Après déprotection, la fonction amine du résidu N-terminal Gly peut par exemple être acétylée par action d'un excès d'anhydride acétique en présence de diisopropyléthylamine.

Les chaînes latérales des acides aminés tri-fonctionnels doivent être protégées notamment par les groupes suivants : le cyclohexyl pour l'acide glutamique et l'acide aspartique, le benzyl pour la thréonine et la sérine, le tosyl pour l'arginine, le paraméthylbenzyl ou l'acétamidométhyl pour la cystéine, le 2,6-dichlorobenzyl ou le 2-bromobenzyloxy-carbonyl pour la tyrosine, le fluorénylméthyloxycarbonyl ou le 2-chlorobenzyloxy-carbonyl pour la lysine, le formyl pour la tryptophane, le benzyloxycarbonyl ou le dinitrophenyl pour l'histidine.

D'autres méthodes peuvent être employées pour la préparation de ces peptides, notamment en protégeant de façon temporaire l'alphamine des acides aminés par le fluorenylméthyl de l'oxycarbonyl.

Après avoir éliminé tous les groupes protecteurs, sauf dans certains cas l'acétamidométhyl de la cystéine, le peptide est libéré du support solide. Le produit brut est lyophylisé et purifié par chromatographie en phase liquide à moyenne pression permettant d'obtenir un peptide pur à environ 70 % ; les produits sont alors purifiés par chromatographie en phase liquide à haute pression (CLHP) ou selon un autre type de chromatographie, permettant d'obtenir le peptide pur à 95 % d'après la CHLP analytique

L'extrémité N-terminale des peptides de l'invention peut comporter un groupe $NH_2$ libre ou amidé par un ou deux groupes alcoyles quelconques ; de même l'extrémité C-terminale peut comporter un groupe OH libre, un groupe amide, ou alcoxyle et contenant alors un groupe alcoyle comprenant de 1 à 5 atomes de carbone.

Outre les peptides précités, l'invention concerne les peptides modifiés par insertion et/ou délétion et/ou substitution d'un ou plusieurs acides aminés, pour autant que les propriétés antigéniques ou immunogènes desdits peptides ne sont pas modifiées, ainsi que ceux dans lesquels la liaison peptidique (-CO-NH-) est remplacée, par exemple, par les structures suivantes :

$$-CO-N(CH_3)-, -CH_2-CH_2-, -CO-CH_2-$$

ou encore dans lesquels le squelette peptidique présente un ou plusieurs groupes intercalés tels que les groupes $-CH_2-$, $-NH-$, $-O-$.

Parmi les modifications précédentes, l'invention vise plus particulièrement les peptides dont le ou les résidus Cys sont supprimés, substitués ou protégés. A titre d'exemple de telles modifications, l'invention s'intéresse davantage aux peptides dans lesquels les résidus Cys sont remplacés par des résidus Ser et ceux dans

3

lesquels le groupement -SH de la chaîne latérale des résidus Cys est protégé par un groupement acétamidométhyl.

La présente invention englobe également les peptides dans lesquels les résidus d'acides aminés présentant un carbone asymétrique sont sous forme Asp ou Leu.

L'invention concerne également les oligomères des monomères constitués par des peptides de l'invention ainsi que la composition antigénique contenant au moins un desdits peptides, ou au moins un oligomère de ce peptide reconnaissant spécifiquement la présence d'anticorps contre l'antigène HBc.

On peut réaliser l'oligomérisation par toute technique de polymérisation d'un peptide, la polymérisation étant effectuée jusqu'à l'obtention d'un oligomère contenant le nombre de monomères permettant d'obtenir l'immunogénicité souhaitée.

L'invention concerne aussi les conjugués obtenus par couplage des peptides de l'invention à des molécules porteuses, ainsi que les compositions immunogènes contenant au moins un desdits peptides ou au moins un oligomère de ce peptide, en association avec un véhicule pharmaceutiquement acceptable, pour la préparation de vaccins induisant la production d'anticorps contre lesdits peptides en quantité suffisante pour inhiber efficacement le virus de l'hépatite B.

A titre d'exemple de molécules porteuses, on peut citer des protéines naturelles comme l'anatoxine tétanique, l'ovalbumine ou des sérum-albumines.

L'invention concerne encore les anticorps formés contre les peptides de l'invention, anticorps qui peuvent être polyclonaux, ou monoclonaux et produits alors par tout hybridome préparé selon les méthodes classiques de fusion cellulaire entre des cellules spléniques d'un animal immunisé contre l'un des peptides de l'invention et des cellules d'une lignée de cellules myélomes. Ces anticorps monoclonaux sont avantageusement dirigés contre l'antigène HBc et reconnaissent immunologiquement un ou plusieurs des peptides de l'invention.

Les peptides de l'invention possèdent des propriétés antigéniques et peuvent donc être utilisés dans des procédés de détection *in vitro* de l'infection par le virus de l'hépatite B, dans un échantillon biologique tel que le sérum humain, comprenant de manière générale :
- la mise en contact de cet échantillon biologique avec au moins un des peptides de l'invention ou un conjugué de ce peptide avec une molécule porteuse ;
- la détection de la présence éventuelle de complexe antigène-anticorps.

La détection des complexes antigène-anticorps éventuellement formés est avantageusement effectuée par des tests immunoenzymatiques, immunofluorescents, radioimmunologiques ou des tests de radioimmunoprécipitation.

L'invention a donc aussi pour objet les peptides précédents marqués notamment par un isotope radioactif, une enzyme, une substance fluorescente, une molécule chargée électriquement, une molécule colorée, la biotine, un composé organo-métallique.

A titre d'exemple des procédés de détection des anticorps contre l'antigène HBc, on peut citer notamment un test immunoenzymatique indirect dans le sérum ou plasma humain comprenant les étapes suivantes :
- dépôt d'une quantité déterminée d'un peptide de l'invention ou d'une composition contenant ledit peptide dans les puits d'une plaque de microtitration ;
- distribution des sérums ou plasma à analyser dilués, ainsi que des sérums de contrôle positifs et négatifs, dans lesdits puits ;
- incubation pendant une durée suffisante pour permettre la fixation des anticorps sériques éventuellement présents aux peptides de l'invention spécifiques du virus de l'hépatite B ;
- lavage de la microplaque ;
- introduction dans les puits d'un conjugué anti-immunoglobuline humaine marqué à la péroxydase se liant aux immunoglobulines du sang retenues sur la phase solide ;
- élimination de la fraction non liée ;
- révélation de la présence éventuelle de l'enzyme par un substrat chromogène ;
- détection et interprétation par comparaison avec les sérums de contrôle.

Parmi les procédés de détection des anticorps contre l'antigène HBc, selon l'invention, on peut citer encore, un test immunoenzymatique indirect rapide comprenant les étapes suivantes :
- dépôt d'une quantité déterminée d'un peptide de l'invention sur une membrane saturée puis séchée ;
- filtration au travers de cette membrane d'un sérum à tester (plasma ou sang total), suivi d'un dépôt d'un conjugué, par exemple, anti-IgG humaine couplée à la péroxydase ;
- après lavage, la réaction est révélée par dépôt d'un substrat chromogène de la péroxydase. Seuls les anticorps anti-antigène HBc fixés aux peptides sont révélés dans cette réaction par un peptide spot. Un témoin interne de validation est inclus dans le test.

La présence d'un spot coloré en position du dépôt préliminaire du peptide ainsi que la présence du contrôle de validation permet l'interprétation positive (présence d'anticorps anti-antigène HBc) pour l'échantillon consi-

EP 0 494 825 A1

déré en un temps de trois minutes environ.

L'invention concerne enfin des nécessaires ou kits pour le diagnostic *in vitro* de l'infection par le virus de l'hépatite B, dans un échantillon biologique, comprenant :

- une quantité donnée d'au moins un peptide de l'invention, ou un mélange de ces peptides, ou un conjugué de ce peptide avec une molécule porteuse ;
- au moins un réactif pour la constitution d'un milieu propice à la réalisation d'une réaction immunologique ;
- un ou plusieurs réactifs éventuellement marqués pour la détection des complexes antigène-anticorps formés lors de la réaction immunologique ;
- au moins un échantillon de contrôle dépourvu d'anticorps ou contenant une quantité connue d'anticorps reconnus par lesdits peptides.

D'autres avantages et caractéristiques de l'invention apparaîtront à la lecture de l'étude qui suit concernant l'utilisation des peptides de l'invention dans un test ELISA indirect pour la détection des anticorps anti-HBc dans des sérums ou plasma humains ; étant entendu que l'exemple de cette étude ne saurait être interprété comme tendant à réduire la portée des revendications.

Cette étude a consisté à tester sur des sérums de réactivité anti-HBc connue, les peptides ou mélanges de peptides suivants :

- peptide de formule (I),
- peptide de formule (II),
- peptide de formule (III),
- peptide de formule (V),
- mélange des peptides de formules (I) et (V),
- mélange des peptides de formules (II) et (V)
- mélange des peptides de formules (I), (II) et (V).

Chacun des peptides ou mélanges de peptides a été utilisé dans un test de diagnostic sérologique sur plaque de microtitration, selon le même protocole suivant :

1) Sensibilisation des plaques de microtitration

- Les peptides sont repris en milieu liquide. Après dilution en tampon à une concentration de 3 µg/ml, les cupules des microplaques sont sensibilisées par 100 µl de cette solution, 16 heures à 37° C ;
- les plaques sont ensuite vidées et 300 µl de solution de saturation sont ajoutés ; incubation 2 heures à la température du laboratoire ;
- après quatre cycles de lavage, les plaques sont séchées à 37° C avant utilisation (stockage en sac étanche avec un dessiccant).

2) Réalisation de la réaction

- Après prélavage de la plaque de microtitration sensibilisée, 100 µl de l'échantillon (sérum de réactivité connue dilué au 1/2) sont déposés par cupule. Des témoins de manipulation sont inclus dans chaque série, l'incubation est effectuée une heure à température ambiante. Durant cette étape les anticorps anti-HBc éventuellement présents dans les échantillons se lient aux peptides de la phase solide ;
- quatre lavages en PBS/TWEEN permettent d'éliminer les anticorps non fixés ;
- distribution de 100 µl d'anticorps anti-immunoglobuline humaine couplés à la péroxydase et l'incubation est effectuée pendant 30 minutes à température ambiante ;
- l'excès de conjugué est éliminé par un nouveau cycle de quatre lavages ;
- le conjugué péroxydase est révélé par 50 µl de substrat OPD (Ortho-phénylènediamine) en présence d'$H_2O_2$ ; cette incubation est conduite pendant 5 minutes à l'obscurité ;
- la réaction de chromogénèse est arrêtée après 5 minutes par 100 µl d'HCl 1N. La densité optique (DO) est lue à 492 nm.

3) Résultats

Une valeur seuil est déterminée à partir des valeurs des sérums de contrôle. Les échantillons dont la densité optique est supérieure à la valeur seuil sont considérés comme positifs.

a) le Tableau 1 ci-dessous rapporte les résultats obtenus avec une population de 31 sérums (déterminés comme positifs pour les anticorps anti-HBc et pour l'antigène HBs dans les tests de référence commercialisés) dans quatre tests ELISA mettant en oeuvre les peptides de formules (V), (I), (II) et (III), pour lesquels les valeurs seuils respectives sont 0,32 ; 0,36 ; 0,47 ; 0,3.

5

TABLEAU 1

| Sérums | Réactivité anti-HBc | Peptide (V) valeur seuil 0,32 | Peptide (I) valeur seuil 0,36 | Peptide (II) valeur seuil 0,47 | Peptide (III) valeur seuil 0,3 |
|---|---|---|---|---|---|
| 1 | + | 0,638 | 0,264 | 0,475 | 0,442 |
| 2 | + | 0,521 | 0,317 | 0,393 | 0,267 |
| 3 | + | 0,164 | 0,148 | 0,148 | 0,152 |
| 4 | + | 0,919 | 2,373 | 0,786 | 0,736 |
| 5 | + | 2,313 | 0,584 | 2,577 | 0,889 |
| 6 | + | 0,925 | 0,429 | 0,554 | 0,327 |
| 7 | + | 0,339 | 0,553 | 0,179 | 0,268 |
| 8 | + | 1,177 | 0,883 | 0,842 | 1,109 |
| 9 | + | 1,068 | 0,313 | 0,934 | 0,316 |
| 10 | + | 0,626 | 0,511 | 0,445 | 0,587 |
| 11 | + | 1,294 | 0,254 | 0,334 | 0,220 |
| 12 | + | 0,822 | 0,346 | 0,815 | 0,444 |
| 13 | + | 0,632 | 0,689 | 0,285 | 0,391 |
| 14 | + | 1,471 | 0,707 | 1,156 | 0,526 |
| 15 | + | 0,485 | 1,169 | 0,243 | 0,083 |
| 16 | + | 0,626 | 0,298 | 0,300 | 0,219 |
| 17 | + | 0,403 | 3,195 | 0,366 | 0,267 |
| 18 | + | 1,092 | 1,374 | 0,829 | 0,743 |
| 19 | + | 2,758 | 0,198 | 2,656 | 0,258 |
| 20 | + | 0,804 | 0,481 | 0,852 | 0,542 |
| 21 | - | 0,229 | 0,173 | 0,168 | 0,224 |
| 22 | - | 0,290 | 0,185 | 0,177 | 0,081 |
| 23 | - | 0,219 | 0,187 | 0,149 | 0,172 |
| 24 | - | 0,220 | 0,236 | 0,438 | 0,185 |
| 25 | - | 0,292 | 0,241 | 0,132 | 0,273 |

| 26 | - | 0,239 | 0,246 | 0,109 | 0,176 |
| 27 | - | 0,229 | 0,191 | 0,181 | 0,200 |
| 28 | - | 0,256 | 0,213 | 0,131 | 0,210 |
| 29 | - | 0,286 | 0,328 | 0,220 | 0,233 |
| 30 | - | 0,259 | 0,234 | 0,141 | 0,210 |
| 31 | - | 0,234 | 0,314 | 0,136 | 0,204 |

b) le Tableau 2 ci-dessous rapporte les résultats obtenus sur une polulation de 35 donneurs de sang anti-HBc positifs en utilisant les peptides de formules (V), (I), (II) et (III), pour lesquels les valeurs seuils respectives sont 0,32 ; 0,36 ; 0,47 ; 0,3.

TABLEAU 2

| Sérums | Réactivité anti-HBc | Peptide (V) valeur seuil 0,32 | Peptide (I) valeur seuil 0,36 | Peptide (II) valeur seuil 0,47 | Peptide (III) valeur seuil 0,3 |
|---|---|---|---|---|---|
| 1 | + | 0,480 | 0,207 | 0,593 | 0,162 |
| 2 | + | 0,328 | 0,261 | 0,197 | 0,196 |
| 3 | - | 0,154 | 0,209 | 0,201 | 0,166 |
| 4 | + | 0,235 | 0,639 | 0,131 | 0,177 |
| 5 | + | 1,108 | 0,614 | 0,152 | 0,112 |
| 6 | + | 2,527 | 1,808 | 0,420 | 0,121 |
| 7 | + | 1,080 | 0,255 | 1,036 | 0,074 |
| 8 | + | 0,288 | 0,243 | 0,243 | 0,151 |
| 9 | + | 0,391 | 0,202 | 0,507 | 0,182 |
| 10 | + | 0,599 | 0,406 | 0,661 | 0,217 |
| 11 | + | 0,675 | 0,884 | 0,793 | 0,152 |
| 12 | + | 0,710 | 0,366 | 0,442 | 0,358 |
| 13 | + | 1,525 | 0,347 | 2,073 | 0,319 |
| 14 | + | 1,475 | 1,167 | 0,415 | 1,179 |
| 15 | + | 1,264 | 0,410 | 0,454 | 0,658 |
| 16 | + | 0,279 | 0,160 | 0,254 | 0,125 |

| | | | | | |
|---|---|---|---|---|---|
| 17 | + | 0,275 | 0,182 | 0,302 | 0,157 |
| 18 | + | 1,193 | 0,202 | 1,045 | 0,191 |
| 19 | + | 0,244 | 0,253 | 0,111 | 2,098 |
| 20 | + | 0,584 | 0,274 | 0,703 | 0,163 |
| 21 | + | 1,946 | 0,711 | 1,859 | 0,277 |
| 22 | + | 0,360 | 0,198 | 0,508 | 0,166 |
| 23 | + | 1,919 | 0,699 | 2,164 | 0,067 |
| 24 | + | 0,258 | 0,183 | 0,156 | 0,204 |
| 25 | - | 0,229 | 0,173 | 0,168 | 0,224 |
| 26 | - | 0,290 | 0,185 | 0,177 | 0,081 |
| 27 | - | 0,219 | 0,187 | 0,149 | 0,172 |
| 28 | - | 0,220 | 0,236 | 0,438 | 0,185 |
| 29 | - | 0,292 | 0,241 | 0,132 | 0,273 |
| 30 | - | 0,239 | 0,246 | 0,109 | 0,176 |
| 31 | - | 0,229 | 0,191 | 0,181 | 0,200 |
| 32 | - | 0,256 | 0,213 | 0,131 | 0,210 |
| 33 | - | 0,286 | 0,328 | 0,220 | 0,233 |
| 34 | - | 0,259 | 0,234 | 0,141 | 0,210 |
| 35 | - | 0,234 | 0,314 | 0,136 | 0,204 |

c) le Tableau 3 ci-dessous rapporte les résultats obtenus sur une population de 32 donneurs de sang anticorps anti-HBc positifs, en utilisant les mélanges des peptides de formules (V) et (I) ; (V) et (II) ; (V), (I) et (II) ; et le peptide de formule (V) ; pour lesquels les valeurs seuils respectives sont 0,33; 0,35; 0,36 ; 0,4.

TABLEAU 3

| Sérums | Réactivité anti-HBc | Peptides (V) et (I) valeur seuil 0,33 | Peptides (V) et (II) valeur seuil 0,35 | Peptides (V),(I) et(II) valeur seuil 0,36 | Peptide (V) valeur seuil 0,4 |
|---|---|---|---|---|---|
| 1 | + | 0,463 | 0,837 | 0,675 | 0,676 |
| 2 | + | 0,330 | 0,441 | 0,326 | 0,391 |
| 3 | + | 0,310 | 0,298 | 0,227 | 0,301 |
| 4 | + | 0,435 | 0,265 | 0,237 | 0,270 |
| 5 | + | 1,509 | 1,054 | 0,684 | 1,208 |
| 6 | + | 2,446 | 2,206 | 1,924 | 2,295 |
| 7 | + | 0,875 | 1,165 | 1,048 | 0,955 |
| 8 | + | 0,368 | 0,392 | 0,387 | 0,301 |
| 9 | + | 0,377 | 0,588 | 0,581 | 0,471 |
| 10 | + | 0,678 | 0,985 | 0,912 | 0,680 |
| 11 | + | 0,924 | 0,925 | 0,898 | 0,772 |
| 12 | + | 0,251 | 0,320 | 0,261 | 0,316 |
| 13 | + | 0,251 | 0,482 | 0,385 | 0,346 |
| 14 | + | 1,159 | 1,492 | 1,557 | 1,136 |
| 15 | + | 0,352 | 0,271 | 0,302 | 0,210 |
| 16 | + | 0,479 | 1,388 | 0,940 | 0,845 |
| 17 | + | 1,745 | 1,991 | 2,295 | 1,993 |
| 18 | + | 0,306 | 0,388 | 0,372 | 0,334 |
| 19 | + | 1,696 | 1,676 | 2,313 | 2,040 |
| 20 | + | 0,515 | 0,640 | 0,464 | 0,832 |
| 21 | + | 0,707 | 0,775 | 0,645 | 0,765 |
| 22 | + | 1,177 | 2,529 | 2,253 | 2,022 |
| 23 | + | 0,744 | 0,944 | 0,765 | 1,170 |
| 24 | + | 1,425 | 1,782 | 1,550 | 1,533 |

| 25 | - | 0,280 | 0,286 | 0,257 | 0,340 |
|----|---|-------|-------|-------|-------|
| 26 | - | 0,223 | 0,160 | 0,165 | 0,228 |
| 27 | - | 0,194 | 0,235 | 0,204 | 0,221 |
| 28 | - | 0,198 | 0,181 | 0,197 | 0,222 |
| 29 | - | 0,266 | 0,226 | 0,228 | 0,271 |
| 30 | - | 0,229 | 0,321 | 0,326 | 0,377 |
| 31 | - | 0,305 | 0,255 | 0,314 | 0,358 |
| 32 | - | 0,199 | 0,202 | 0,223 | 0,229 |

**Revendications**

1.  Peptides synthétiques constitués par tout ou partie d'au moins une des séquences peptidiques choisie parmi les séquences suivantes :

```
Leu-Tyr-Arg-Glu-Ala-Leu-Glu-Ser-Pro-Glu-His-Cys-Ser-Pro-
His-His-Thr-Ala-Leu-Arg-Gln-Ala
Val-Asn-Leu-Glu-Asp-Pro--Ala-Ser-Arg-Asp-Leu-Val-Val-Ser-
Tyr-Val-Asn-Thr-Asn-Met-Gly-Leu-Lys-Phe-Arg-Gln-Leu
Lys-Phe-Arg-Gln-Leu-Leu-Trp-Phe-His-Ile-Ser-Cys-Leu-Thr-
Phe-Gly-Arg-Glu-Thr-Val-Ile-Glu-Tyr
Gly-Arg-Ser-Pro-Arg-Arg-Arg-Thr-Pro-Ser-Pro-Arg-Arg-Arg-
Arg-Ser-Gln-Ser-Pro-Arg-Arg-Arg-Arg-Ser-Gln-Ser-Arg-Glu-
Ser-Gln-Cys
Val-Asn-Leu-Glu-Asp-Pro-Ala-Ser-Arg-Asp-Leu-Val-Val-Ser-
Tyr-Val-Asn-Thr-Asn-Met-Gly-Leu-Lys-Phe-Arg-Gln-Leu-Leu-
Trp-Phe-His-Ile-Ser-Cys-Leu-Thr-Phe-Gly-Arg-Glu-Thr-Val-
Ile-Glu-Tyr
```

2.  Peptide selon la revendication 1, répondant à la formule suivante :

```
Leu-Tyr-Arg-Glu-Ala-Leu-Glu-Ser-Pro-Glu-His-Cys-Ser-Pro-
His-His-Thr-Ala-Leu-Arg-Gln-Ala                        (I)
```

3.  Peptide selon la revendication 1, répondant à la formule suivante :

```
Val-Asn-Leu-Glu-Asp-Pro--Ala-Ser-Arg-Asp-Leu-Val-Val-Ser-
Tyr-Val-Asn-Thr-Asn-Met-Gly-Leu-Lys-Phe-Arg-Gln-Leu (II)
```

4. Peptide selon la revendication 1, répondant à la formule suivante :

```
Lys-Phe-Arg-Gln-Leu-Leu-Trp-Phe-His-Ile-Ser-Cys-Leu-Thr-
Phe-Gly-Arg-Glu-Thr-Val-Ile-Glu-Tyr                    (III)
```

5. Peptide selon la revendication 1, répondant à la formule suivante :

```
Gly-Arg-Ser-Pro-Arg-Arg-Arg-Thr-Pro-Ser-Pro-Arg-Arg-Arg-
Arg-Ser-Gln-Ser-Pro-Arg-Arg-Arg-Arg-Ser-Gln-Ser-Arg-Glu-
Ser-Gln-Cys                                            (IV)
```

6. Peptide selon la revendication 1, répondant à la formule suivante :

```
Val-Asn-Leu-Glu-Asp-Pro-Ala-Ser-Arg-Asp-Leu-Val-Val-Ser-
Tyr-Val-Asn-Thr-Asn-Met-Gly-Leu-Lys-Phe-Arg-Gln-Leu-Leu-
Trp-Phe-His-Ile-Ser-Cys-Leu-Thr-Phe-Gly-Arg-Glu-Thr-Val-
Ile-Glu-Tyr                                            (V)
```

7. Peptide selon l'une quelconque des revendications 1 à 6, caractérisé en ce qu'au moins un des résidus Cys est délété , substitué ou protégé.

8. Peptide selon la revendication 7, caractérisé en ce qu'au moins un des résidus Cys est substitué par un résidu Ser.

9. Peptide selon la revendication 7, caractérisé en ce que la chaîne latérale d'au moins un des résidus Cys est protégée par un groupement acétamidométhyl.

10. Peptide selon l'une quelconque des revendications 1 à 9, caractérisé en ce que son extrémité N-terminale comporte un groupe -NH$_2$ libre ou amidé par un ou deux groupes alcoyles comprenant de 1 à 5 atomes de carbone, et son extrémité C-terminale comporte un groupe -OH libre ou alcoxyle et contenant alors un groupe alcoyle comprenant de 1 à 5 atomes de carbone.

11. Composition immunogène contenant au moins un peptide selon l'une quelconque des revendications 1 à 10 ou au moins un oligomère de ce peptide ou ce peptide conjugué à une molécule porteuse, en association avec un véhicule pharmaceutiquement acceptable pour la production de vaccins, caractérisée en ce qu'elle induit la production d'anticorps contre lesdits peptides en quantité suffisante pour inhiber efficacement le virus de l'hépatite B.

12. Anticorps monoclonaux dirigés contre l'antigène HBc, caractérisés en ce qu'ils reconnaissent immunologiquement un ou plusieurs des peptides selon l'une quelconque des revendications 1 à 10.

13. Procédé de diagnostic *in vitro* de l'infection par le virus de l'hépatite B, dans un échantillon biologique caractérisé en ce qu'il comprend :
    −la mise en contact de cet échantillon avec au moins un peptide selon l'une quelconque des revendications 1 à 10 ou un conjugué de ce peptide avec une molécule porteuse ,
    −la détection de la présence éventuelle de complexe antigène-anticorps.

14. Procédé de diagnostic *in vitro* de l'infection par le virus de l'hépatite B, dans un échantillon biologique, selon la revendication 13, caractérisé en ce que la détection des complexes antigènes-anticorps éventuellement formés est effectuée par des tests immunoenzymatiques, radioimmunologiques ou des tests de radioimmunoprécipitation.

15. Nécessaire pour la mise en oeuvre d'un procédé de diagnostic *in vitro* de l'infection par le virus de l'hépatite B, dans un échantillon biologique selon la revendication 13, caractérisé en ce qu'il comprend :
- une composition peptidique contenant au moins un peptide selon l'une des revendications 1 à 10, ou un conjugué de ce peptide avec une molécule porteuse,
- au moins un réactif pour la constitution d'un milieu propice à la réalisation d'une réaction immunologique ;
- un ou plusieurs réactifs éventuellement marqués pour la détection des complexes antigène-anticorps formés lors de la réaction immunologique ;
- au moins un échantillon de référence dépourvu d'anticorps ou contenant une quantité connue d'anticorps reconnus par ladite composition peptidique.

Office européen
des brevets

## RAPPORT DE RECHERCHE EUROPEENNE

Numéro de la demande

EP 92 40 0053

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 271 302 (SCRIPPS CLINIC AND RESEARCH FOUNDATION) <br> * Document en entier * <br> --- | 1-4,6,7 -15 | C 07 K 7/10 <br> C 12 P 21/08 <br> A 61 K 39/29 <br> G 01 N 33/576 |
| X | JOURNAL OF IMMUNOLOGY, vol. 139, no. 4, 15 août 1987, BALTIMORE, US, pages 1223 - 1231; MILICH C.S.: 'Immune response to hepatitis B virus core antigen (HBcAg): Localization of T cell recognition sites within HBcAg/HBeAg' <br> * Le document en entier; spec. figures 5,6 * <br> --- | 1-15 | |
| X | JOURNAL OF IMMUNOLOGY, vol. 141, no. 12, 15 décembre 1988, BALTIMORE, US, pages 4376-4380; COLUCCI C.S.: 'Identification of a major hepatitis B core antigen (HBcAg) determinant by using synthetic peptides and monoclonal antibodies' <br> * Le document en entier * <br> --- | 1-15 | |
| X | VACCINE, vol. 6, no. 2, avril 1988, GUILDFORD, GB, pages 164-174; MURRAY K.: 'Application of recombinant DNA techniques in the development of viral vaccines' <br> * Le document en entier * <br> --- | 1-15 | **DOMAINES TECHNIQUES RECHERCHES (Int. Cl.5)** <br><br> C 07 K <br> A 61 K <br> C 12 P <br> G 01 N <br> C 12 N |
| X | MOLECULAR IMMUNOLOGY vol. 26, no. 4, avril 1989, LONDON, GB, pages 413-421; MACHIDA C.S.: 'Antigenic sites on the arginine rich carboxyl-terminal domain of the capsid protein of hepatitis B virus distinct from hepatitis B core or e antigen' <br> * Le document en entier * <br> ---       -/- | 1,5,7- 15 | |

Le présent rapport a été établi pour toutes les revendications

| Lieu de la recherche | Date d'achèvement de la recherche | Examinateur |
|---|---|---|
| LA HAYE | 27-03-1992 | GROENENDIJK M.S.M. |

Office européen

des brevets

**RAPPORT DE RECHERCHE EUROPEENNE**

Numéro de la demande

EP  92 40 0053

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 192 811  (CETUS CORPORATION) <br> * Page 5, ligne 24 - page 6, ligne 26; revendications 1-3 * | 7-9 | |
| A | EP-A-0 355 460  (AMERICAN CYANAMID CORPORATION) <br> * Page 2, ligne 4 - page 3, ligne 21; revendications 1-6 * | 7-9 | |

**DOMAINES TECHNIQUES RECHERCHES (Int. Cl.5)**

Le présent rapport a été établi pour toutes les revendications

| Lieu de la recherche | Date d'achèvement de la recherche | Examinateur |
|---|---|---|
| LA HAYE | 27-03-1992 | GROENENDIJK M.S.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)